# EUROPEAN PATENT APPLICATION

(11) **EP 1 132 474 A1**
(43) Date of publication of application: **12.09.2001**
(21) Application number: 01104583.8
(22) Date of filing: 06.03.2001
(51) Int. Cl.: C12N 15/52, C12N 9/00

(54) **Mutant B-typ DNA polymerases exhibiting improved performance in PCR**

(30) Priority: 11.03.2000 EP 00105155
(71) Applicant: Roche Diagnostics GmbH, 68298 Mannheim (DE)
(72) Inventor: Sobek, Harald Dr., 82377 Penzberg (DE); Frey, Bruno Dr., 82377 Penzberg (DE); Antranikian, Garabed Prof. Dr., 21218 Seevetal-Hittfeld (DE); Boehlke, Kristina, 22761 Hamburg (DE); Pisani, Francesca Maria Dr., 00140 Rom (IT); Rossi, Mosè Prof. Dr., 80072 Arcofelice/Napoli (IT)

(57) **Abstract**

Claimed are thermostable mutants of B-type DNA polymerases having a Y-GG/A amino acid motif between the N-terminal 3'-5'-exonuclease domain and the C-terminal polymerase domain whereas the tyrosine of the Y-GG/A amino acid motif is mutated and whereas these mutant DNA polymerases are suitable for PCR.

SEQUENCE LISTING

<110> Roche Diagnostics GmbH

<120> Mutant B-type DNA polymerase exhibiting improved
      performance in PCR

<130> 5328/00/EP

<140> 00105115.6
<141> 2000-03-11

<160> 34

<170> PatentIn Ver. 2.1

<210> 1
<211> 35
<212> DNA
<213> Artificial Sequence

<220>
<223> Description of Artificial Sequence: Artificial

<400> 1
gcaaccttgt agagtagagt ggtacctgtt aaggg                                35

<210> 2
<211> 44
<212> DNA
<213> Artificial Sequence

<220>
<223> Description of Artificial Sequence: Artificial

<400> 2
gcctctttcc ggctctttta cgtatcctcc caggaaagta gtcc                      44

<210> 3
<211> 44
<212> DNA
<213> Artificial Sequence

<220>
<223> Description of Artificial Sequence: Artificial

<400> 3
gcctctttcc ggctctttta cgtatcctcc caggtgagta gtcc                      44

<210> 4
<211> 44
<212> DNA
<213> Artificial Sequence

<220>
<223> Description of Artificial Sequence: Artificial

<400> 4
gcctctttcc ggctctttta cgtatcctcc caggttagta gtcc                44

<210> 5
<211> 44
<212> DNA
<213> Artificial Sequence

<220>
<223> Description of Artificial Sequence: Artificial

<400> 5
gcctctttcc ggctctttta cgtatcctcc cagggaagta gtcc                44

<210> 6
<211> 44
<212> DNA
<213> Artificial Sequence

<220>
<223> Description of Artificial Sequence: Artificial

<400> 6
gcctctttcc ggctctttta cgtatcctcc cagccaagta gtcc                44

<210> 7
<211> 44
<212> DNA
<213> Artificial Sequence

<220>
<223> Description of Artificial Sequence: Artificial

<400> 7
gcctctttcc ggctctttta cgtatccagc caggtaagta gtcc                44

<210> 8
<211> 34
<212> DNA
<213> Artificial Sequence

<220>
<223> Description of Artificial Sequence: Artificial

<400> 8
agcttatcga tggcactttt cggggaaatg tgcg                34

<210> 9
<211> 36
<212> DNA
<213> Artificial Sequence

<220>
<223> Description of Artificial Sequence: Artificial

<400> 9
agcttatcga taagcggatg ccgggagcag acaagc                                36

<210> 10
<211> 23
<212> DNA
<213> Artificial Sequence

<220>
<223> Description of Artificial Sequence: Artificial

<400> 10
gatgagttcg tgtccgtaca aca                                              23

<210> 11
<211> 23
<212> DNA
<213> Artificial Sequence

<220>
<223> Description of Artificial Sequence: Artificial

<400> 11
ctcatcagca gatcatcttc agg                                              23

<210> 12
<211> 23
<212> DNA
<213> Artificial Sequence

<220>
<223> Description of Artificial Sequence: Artificial

<400> 12
actccagcgt ctcatcttta tgc                                              23

<210> 13
<211> 23
<212> DNA
<213> Artificial Sequence

<220>
<223> Description of Artificial Sequence: Artificial

<400> 13
gatggtgatc ctctctcgtt tgc                                              23

<210> 14
<211> 23
<212> DNA
<213> Artificial Sequence

<220>
<223> Description of Artificial Sequence: Artificial

<400> 14
gatgagttcg tgtccgtaca aca                                              23

<210> 15
<211> 23
<212> DNA
<213> Artificial Sequence

<220>
<223> Description of Artificial Sequence: Artificial

<400> 15
cttcatcatc gagatagctg tcg                                              23

<210> 16
<211> 25
<212> PRT
<213> T. aggregans

<400> 16
Glu Tyr Arg Arg Arg Leu Arg Thr Thr Tyr Leu Gly Gly Tyr Val Lys
  1               5                  10                  15

Glu Pro Glu Arg Gly Leu Trp Glu Asn
             20                  25

<210> 17
<211> 25
<212> PRT
<213> T. litoralis

<400> 17
Glu Tyr Lys Arg Arg Leu Arg Thr Thr Tyr Leu Gly Gly Tyr Val Lys
  1               5                  10                  15

Glu Pro Glu Lys Gly Leu Trp Glu Asn
             20                  25

<210> 18
<211> 25
<212> PRT
<213> T. fumicolans

<400> 18
Glu Leu Glu Arg Arg Xaa Arg Gly Gly Tyr Ala Gly Gly Tyr Val Lys
1               5                   10                  15

Glu Pro Glu Arg Gly Leu Trp Glu Asn
            20                  25

<210> 19
<211> 25
<212> PRT
<213> T. spec. 9N7

<400> 19
Glu Leu Ala Arg Arg Xaa Arg Gly Gly Tyr Ala Gly Gly Tyr Val Lys
1               5                   10                  15

Glu Arg Glu Arg Gly Leu Trp Glu Asn
            20                  25

<210> 20
<211> 25
<212> PRT
<213> T. gorgonarius

<400> 20
Glu Leu Ala Arg Arg Xaa Arg Glu Ser Tyr Ala Gly Gly Tyr Val Lys
1               5                   10                  15

Glu Pro Glu Arg Gly Leu Trp Glu Asn
            20                  25

<210> 21
<211> 25
<212> PRT
<213> P. spec. KOD

<400> 21
Glu Leu Ala Arg Arg Xaa Arg Gln Ser Tyr Glu Gly Gly Tyr Val Lys
1               5                   10                  15

Glu Pro Glu Arg Gly Leu Trp Glu Asn
            20                  25

<210> 22
<211> 25
<212> PRT
<213> P. abysii

<400> 22
Glu Tyr Glu Arg Arg Leu Arg Glu Ser Tyr Glu Gly Gly Tyr Val Lys
1               5                   10                  15

Glu Pro Glu Lys Gly Leu Trp Glu Asn
            20                  25


<210> 23
<211> 25
<212> PRT
<213> P. furiosus

<400> 23
Glu Tyr Gln Arg Arg Leu Arg Glu Ser Tyr Thr Gly Gly Phe Val Lys
1               5                   10                  15

Glu Pro Glu Lys Gly Leu Trp Glu Asn
            20                  25


<210> 24
<211> 25
<212> PRT
<213> P. horikoshii

<400> 24
Glu Tyr Glu Arg Arg Leu Arg Glu Ser Tyr Glu Gly Gly Tyr Val Lys
1               5                   10                  15

Glu Pro Glu Lys Gly Leu Trp Glu Asn
            20                  25


<210> 25
<211> 25
<212> PRT
<213> M. jannaschii

<400> 25
Glu Tyr Arg Arg Arg Val Leu Thr Thr Tyr Glu Gly Gly Tyr Val Lys
1               5                   10                  15

Glu Pro Glu Lys Gly Met Phe Glu Asp
            20                  25

<210> 26
<211> 25
<212> PRT
<213> M. voltae

<400> 26
Ser Tyr Arg Glu Arg Ala Lys Phe Ser Tyr Glu Gly Gly Tyr Val Arg
1               5                   10                  15

Glu Pro Leu Lys Gly Ile Gln Glu Asn
            20                  25

<210> 27
<211> 25
<212> PRT
<213> S. solfataricus

<400> 27
Thr Ser Ala Leu Ile Lys Gly Lys Gly Tyr Lys Gly Ala Val Val Ile
1               5                   10                  15

Asp Pro Pro Ala Gly Ile Phe Phe Asn
            20                  25

<210> 28
<211> 25
<212> PRT
<213> S. acidocaldarius

<400> 28
Thr Ala Ala Val Ile Lys Gly Lys Lys Tyr Lys Gly Ala Val Val Ile
1               5                   10                  15

Asp Pro Pro Ala Gly Val Tyr Phe Asn
            20                  25

<210> 29
<211> 25
<212> PRT
<213> P. islandicum

<400> 29
Thr Lys Ala Ile Ile Lys Gly Lys Lys Tyr Ala Gly Ala Val Val Leu
1               5                   10                  15

Asp Pro Pro Leu Gly Ile Phe Phe Asn
            20                  25

<210> 30
<211> 25
<212> PRT
<213> P. occultum

<400> 30
Ser Glu Ala Leu Ile Lys Gly Lys Lys Tyr Gln Gly Ala Leu Val Leu
1               5                   10                  15

Asp Pro Pro Ser Gly Ile Tyr Phe Asn
            20                  25

<210> 31
<211> 25
<212> PRT
<213> A. pernix

<400> 31
Val Gly Ala Ile Ile Lys Asp Lys Lys Tyr Arg Gly Ala Ile Val Leu
1               5                   10                  15

Asp Pro Pro Val Gly Ile Phe Phe Arg
            20                  25

<210> 32
<211> 25
<212> PRT
<213> S. chwakuensis

<400> 32
Thr Ala Ala Ile Ser Lys Gly Lys Arg Tyr Lys Gly Ala Val Val Ile
1               5                   10                  15

Asp Pro Pro Ala Gly Val Phe Phe Asn
            20                  25

<210> 33
<211> 2325
<212> DNA
<213> T. aggregans

<220>
<221> CDS
<222> (1)..(2325)

<400> 33
atg ata ttt gac act gac tac ata aca aag gac ggt aaa ccc ata att     48
Met Ile Phe Asp Thr Asp Tyr Ile Thr Lys Asp Gly Lys Pro Ile Ile
1               5                   10                  15

cga att ttc aag aaa gag aac ggg gaa ttt aaa ata gaa ctt gat cca 96
Arg Ile Phe Lys Lys Glu Asn Gly Glu Phe Lys Ile Glu Leu Asp Pro
            20                  25                  30

cat ttt cag ccc tac att tac gct ctt ctc aaa gat gac tcc gct att 144
His Phe Gln Pro Tyr Ile Tyr Ala Leu Leu Lys Asp Asp Ser Ala Ile
        35                  40                  45

gat gaa ata aaa gca ata aaa ggc gag aga cac gga aaa att gtg aga 192
Asp Glu Ile Lys Ala Ile Lys Gly Glu Arg His Gly Lys Ile Val Arg
    50                  55                  60

gta gtc gat gca gtg aaa gtc aag aag aaa ttt ttg ggg aga gat gtt 240
Val Val Asp Ala Val Lys Val Lys Lys Lys Phe Leu Gly Arg Asp Val
65                  70                  75                  80

gag gtc tgg aag ctt ata ttt gag cat ccc caa gac gtc ccg gcc cta 288
Glu Val Trp Lys Leu Ile Phe Glu His Pro Gln Asp Val Pro Ala Leu
                85                  90                  95

agg ggc aag ata agg gaa cat cca gct gtg att gac att tat gag tac 336
Arg Gly Lys Ile Arg Glu His Pro Ala Val Ile Asp Ile Tyr Glu Tyr
            100                 105                 110

gac ata ccc ttt gcc aag cgc tac ctc ata gac aag ggc ttg atc cct 384
Asp Ile Pro Phe Ala Lys Arg Tyr Leu Ile Asp Lys Gly Leu Ile Pro
        115                 120                 125

atg gag ggc gac gag gag ctt aag cta atg gcc ttc gac att gag acg 432
Met Glu Gly Asp Glu Glu Leu Lys Leu Met Ala Phe Asp Ile Glu Thr
    130                 135                 140

ttt tac cac gag gga gac gag ttt ggg aag ggc gag ata ata atg ata 480
Phe Tyr His Glu Gly Asp Glu Phe Gly Lys Gly Glu Ile Ile Met Ile
145                 150                 155                 160

agc tac gcc gat gag gaa gag gca agg gta att aca tgg aag aat att 528
Ser Tyr Ala Asp Glu Glu Glu Ala Arg Val Ile Thr Trp Lys Asn Ile
                165                 170                 175

gat ctg ccc tac gtt gat gtt gta tcc aac gaa agg gag atg ata aag 576
Asp Leu Pro Tyr Val Asp Val Val Ser Asn Glu Arg Glu Met Ile Lys
            180                 185                 190

cgg ttt gtg caa att gtc agg gaa aaa gac ccg gat gtc ctg ata act 624
Arg Phe Val Gln Ile Val Arg Glu Lys Asp Pro Asp Val Leu Ile Thr
        195                 200                 205

tac aat gga gac aac ttt gat ttg ccg tac ctt ata aaa agg gca gag 672
Tyr Asn Gly Asp Asn Phe Asp Leu Pro Tyr Leu Ile Lys Arg Ala Glu
    210                 215                 220

aag tta gga gtt act ctt ctc ttg ggg agg gac aaa gaa cac ccc gag     720
Lys Leu Gly Val Thr Leu Leu Leu Gly Arg Asp Lys Glu His Pro Glu
225                 230                 235                 240

ccc aag att cac aga atg ggc gat agc ttt gcc gtg gaa att aaa ggc     768
Pro Lys Ile His Arg Met Gly Asp Ser Phe Ala Val Glu Ile Lys Gly
                245                 250                 255

aga att cac ttt gat ctc ttc ccg gtt gtg cgg aga acc ata aac ctc     816
Arg Ile His Phe Asp Leu Phe Pro Val Val Arg Arg Thr Ile Asn Leu
            260                 265                 270

cca aca tac acg ctt gag gca gtt tat gaa gcc gtc ttg gga aaa acc     864
Pro Thr Tyr Thr Leu Glu Ala Val Tyr Glu Ala Val Leu Gly Lys Thr
        275                 280                 285

aaa agc aag ctg ggt gcg gag gaa atc gcc gcc atc tgg gaa aca gag     912
Lys Ser Lys Leu Gly Ala Glu Glu Ile Ala Ala Ile Trp Glu Thr Glu
    290                 295                 300

gag agc atg aag aag ctg gcc cag tac tcg atg gaa gat gct agg gca     960
Glu Ser Met Lys Lys Leu Ala Gln Tyr Ser Met Glu Asp Ala Arg Ala
305                 310                 315                 320

act tat gaa ctc gga aaa gag ttt ttc ccc atg gag gca gag cta gca     1008
Thr Tyr Glu Leu Gly Lys Glu Phe Phe Pro Met Glu Ala Glu Leu Ala
                325                 330                 335

aag cta ata ggc caa agc gta tgg gac gtc tca aga tca agc act ggc     1056
Lys Leu Ile Gly Gln Ser Val Trp Asp Val Ser Arg Ser Ser Thr Gly
            340                 345                 350

aac ctt gta gag tgg tac ctg tta agg gtg gca tat gag agg aat gag     1104
Asn Leu Val Glu Trp Tyr Leu Leu Arg Val Ala Tyr Glu Arg Asn Glu
        355                 360                 365

ctc gct ccg aac aag ccg gat gaa gaa gag tac aga agg cgt tta agg     1152
Leu Ala Pro Asn Lys Pro Asp Glu Glu Glu Tyr Arg Arg Arg Leu Arg
    370                 375                 380

act act tac ctg gga gga tac gta aaa gag ccg gaa aga ggc tta tgg     1200
Thr Thr Tyr Leu Gly Gly Tyr Val Lys Glu Pro Glu Arg Gly Leu Trp
385                 390                 395                 400

gag aac atc acc tat tta gac ttt agg tgc cta tac ccc tca att ata     1248
Glu Asn Ile Thr Tyr Leu Asp Phe Arg Cys Leu Tyr Pro Ser Ile Ile
                405                 410                 415

gtt acc cac aac gtc tcc cct gac act tta gaa aga gaa ggc tgc aag     1296
Val Thr His Asn Val Ser Pro Asp Thr Leu Glu Arg Glu Gly Cys Lys
            420                 425                 430

aat tac gat gtt gcc ccg ata gta ggt tat aag ttc tgc aag gat ttt     1344
Asn Tyr Asp Val Ala Pro Ile Val Gly Tyr Lys Phe Cys Lys Asp Phe
        435                 440                 445

ccc ggt ttc att cca tct ata ctc ggg gaa tta atc aca atg agg caa     1392
Pro Gly Phe Ile Pro Ser Ile Leu Gly Glu Leu Ile Thr Met Arg Gln
    450                 455                 460

gaa ata aag aag aag atg aaa gct aca att gac cca ata gaa aag aaa     1440
Glu Ile Lys Lys Lys Met Lys Ala Thr Ile Asp Pro Ile Glu Lys Lys
465                 470                 475                 480

atg ctt gat tat agg caa aga gct gtt aaa ttg cac gca aac agc tat     1488
Met Leu Asp Tyr Arg Gln Arg Ala Val Lys Leu His Ala Asn Ser Tyr
                485                 490                 495

tac ggt tat atg ggc tat ccc aag gcg agg tgg tac tcg aag gaa tgt     1536
Tyr Gly Tyr Met Gly Tyr Pro Lys Ala Arg Trp Tyr Ser Lys Glu Cys
            500                 505                 510

gcc gaa agc gtt acc gcg tgg gga agg cac tac ata gaa atg acc ata     1584
Ala Glu Ser Val Thr Ala Trp Gly Arg His Tyr Ile Glu Met Thr Ile
        515                 520                 525

aaa gag ata gag gag aaa ttt gga ttt aag gtg cta tat gcc gac act     1632
Lys Glu Ile Glu Glu Lys Phe Gly Phe Lys Val Leu Tyr Ala Asp Thr
    530                 535                 540

gat ggt ttt tac gcc aca ata ccg gga gaa aaa cct gaa aca atc aaa     1680
Asp Gly Phe Tyr Ala Thr Ile Pro Gly Glu Lys Pro Glu Thr Ile Lys
545                 550                 555                 560

aag aaa gct aag gaa ttc tta aaa tac ata aac tcc aaa ctt ccc ggt     1728
Lys Lys Ala Lys Glu Phe Leu Lys Tyr Ile Asn Ser Lys Leu Pro Gly
                565                 570                 575

ctg ctc gag ctt gag tat gag ggc ttt tac ttg aga gga ttt ttc gtc     1776
Leu Leu Glu Leu Glu Tyr Glu Gly Phe Tyr Leu Arg Gly Phe Phe Val
            580                 585                 590

gca aag aag cgc tat gcg gtt ata gac gaa gaa ggt agg ata acg aca     1824
Ala Lys Lys Arg Tyr Ala Val Ile Asp Glu Glu Gly Arg Ile Thr Thr
        595                 600                 605

agg ggt ctg gaa gtt gta agg agg gac tgg agc gaa ata gcc aaa gag     1872
Arg Gly Leu Glu Val Val Arg Arg Asp Trp Ser Glu Ile Ala Lys Glu
    610                 615                 620

acc cag gct aaa gtc ttg gag gca ata ctt aaa gaa gat agt gtc gaa     1920
Thr Gln Ala Lys Val Leu Glu Ala Ile Leu Lys Glu Asp Ser Val Glu
625                 630                 635                 640

aaa gct gtg gaa atc gtt aag gac gtt gtt gag gag ata gca aaa tac     1968
Lys Ala Val Glu Ile Val Lys Asp Val Val Glu Glu Ile Ala Lys Tyr
                645                 650                 655

caa gtc ccg ctt gaa aag ctt gtt atc cac gag cag att acc aag gat     2016
Gln Val Pro Leu Glu Lys Leu Val Ile His Glu Gln Ile Thr Lys Asp
            660                 665                 670

cta agt gaa tac aaa gcc att ggg cct cat gta gca ata gca aag agg     2064
Leu Ser Glu Tyr Lys Ala Ile Gly Pro His Val Ala Ile Ala Lys Arg
        675                 680                 685

ctt gct gca aag gga ata aaa gtg aga ccc ggc acg ata ata agc tat     2112
Leu Ala Ala Lys Gly Ile Lys Val Arg Pro Gly Thr Ile Ile Ser Tyr
    690                 695                 700

atc gtc ctc agg gga agc gga aag ata agt gac agg gta att ttg ctt     2160
Ile Val Leu Arg Gly Ser Gly Lys Ile Ser Asp Arg Val Ile Leu Leu
705                 710                 715                 720

tca gag tat gat ccg aaa aaa cac aag tac gac ccc gac tac tac ata     2208
Ser Glu Tyr Asp Pro Lys Lys His Lys Tyr Asp Pro Asp Tyr Tyr Ile
                725                 730                 735

gaa aac caa gtt ctg ccg gcg gtg ctt agg atc ctt gaa gcc ttc ggc     2256
Glu Asn Gln Val Leu Pro Ala Val Leu Arg Ile Leu Glu Ala Phe Gly
            740                 745                 750

tac aga aaa gag gac tta aaa tac caa agc tca aaa cag gtt gga ctg     2304
Tyr Arg Lys Glu Asp Leu Lys Tyr Gln Ser Ser Lys Gln Val Gly Leu
        755                 760                 765

gac gcg tgg ctt aag aag tag                                         2325
Asp Ala Trp Leu Lys Lys
    770                 775


<210> 34
<211> 775
<212> PRT
<213> T. aggregans

<400> 34
Met Ile Phe Asp Thr Asp Tyr Ile Thr Lys Asp Gly Lys Pro Ile Ile
1               5                   10                  15

Arg Ile Phe Lys Lys Glu Asn Gly Glu Phe Lys Ile Glu Leu Asp Pro
            20                  25                  30

His Phe Gln Pro Tyr Ile Tyr Ala Leu Leu Lys Asp Asp Ser Ala Ile
        35                  40                  45

Asp Glu Ile Lys Ala Ile Lys Gly Glu Arg His Gly Lys Ile Val Arg
50                  55                  60

Val Val Asp Ala Val Lys Val Lys Lys Lys Phe Leu Gly Arg Asp Val
65                  70                  75                  80

Glu Val Trp Lys Leu Ile Phe Glu His Pro Gln Asp Val Pro Ala Leu
                85                  90                  95

Arg Gly Lys Ile Arg Glu His Pro Ala Val Ile Asp Ile Tyr Glu Tyr
            100                 105                 110

Asp Ile Pro Phe Ala Lys Arg Tyr Leu Ile Asp Lys Gly Leu Ile Pro
        115                 120                 125

Met Glu Gly Asp Glu Glu Leu Lys Leu Met Ala Phe Asp Ile Glu Thr
    130                 135                 140

Phe Tyr His Glu Gly Asp Glu Phe Gly Lys Gly Glu Ile Ile Met Ile
145                 150                 155                 160

Ser Tyr Ala Asp Glu Glu Glu Ala Arg Val Ile Thr Trp Lys Asn Ile
                165                 170                 175

Asp Leu Pro Tyr Val Asp Val Val Ser Asn Glu Arg Glu Met Ile Lys
            180                 185                 190

Arg Phe Val Gln Ile Val Arg Glu Lys Asp Pro Asp Val Leu Ile Thr
        195                 200                 205

Tyr Asn Gly Asp Asn Phe Asp Leu Pro Tyr Leu Ile Lys Arg Ala Glu
    210                 215                 220

Lys Leu Gly Val Thr Leu Leu Leu Gly Arg Asp Lys Glu His Pro Glu
225                 230                 235                 240

Pro Lys Ile His Arg Met Gly Asp Ser Phe Ala Val Glu Ile Lys Gly
                245                 250                 255

Arg Ile His Phe Asp Leu Phe Pro Val Val Arg Arg Thr Ile Asn Leu
            260                 265                 270

Pro Thr Tyr Thr Leu Glu Ala Val Tyr Glu Ala Val Leu Gly Lys Thr
        275                 280                 285

Lys Ser Lys Leu Gly Ala Glu Glu Ile Ala Ala Ile Trp Glu Thr Glu
    290                 295                 300

Glu Ser Met Lys Lys Leu Ala Gln Tyr Ser Met Glu Asp Ala Arg Ala
305                 310                 315                 320

Thr Tyr Glu Leu Gly Lys Glu Phe Phe Pro Met Glu Ala Glu Leu Ala
                325                 330                 335

Lys Leu Ile Gly Gln Ser Val Trp Asp Val Ser Arg Ser Ser Thr Gly
                340                 345                 350

Asn Leu Val Glu Trp Tyr Leu Leu Arg Val Ala Tyr Glu Arg Asn Glu
        355                 360                 365

Leu Ala Pro Asn Lys Pro Asp Glu Glu Glu Tyr Arg Arg Arg Leu Arg
    370                 375                 380

Thr Thr Tyr Leu Gly Gly Tyr Val Lys Glu Pro Glu Arg Gly Leu Trp
385                 390                 395                 400

Glu Asn Ile Thr Tyr Leu Asp Phe Arg Cys Leu Tyr Pro Ser Ile Ile
                405                 410                 415

Val Thr His Asn Val Ser Pro Asp Thr Leu Glu Arg Glu Gly Cys Lys
                420                 425                 430

Asn Tyr Asp Val Ala Pro Ile Val Gly Tyr Lys Phe Cys Lys Asp Phe
        435                 440                 445

Pro Gly Phe Ile Pro Ser Ile Leu Gly Glu Leu Ile Thr Met Arg Gln
    450                 455                 460

Glu Ile Lys Lys Lys Met Lys Ala Thr Ile Asp Pro Ile Glu Lys Lys
465                 470                 475                 480

Met Leu Asp Tyr Arg Gln Arg Ala Val Lys Leu His Ala Asn Ser Tyr
                485                 490                 495

Tyr Gly Tyr Met Gly Tyr Pro Lys Ala Arg Trp Tyr Ser Lys Glu Cys
                500                 505                 510

Ala Glu Ser Val Thr Ala Trp Gly Arg His Tyr Ile Glu Met Thr Ile
        515                 520                 525

Lys Glu Ile Glu Glu Lys Phe Gly Phe Lys Val Leu Tyr Ala Asp Thr
    530                 535                 540

Asp Gly Phe Tyr Ala Thr Ile Pro Gly Glu Lys Pro Glu Thr Ile Lys
545                 550                 555                 560

Lys Lys Ala Lys Glu Phe Leu Lys Tyr Ile Asn Ser Lys Leu Pro Gly
                565                 570                 575

Leu Leu Glu Leu Glu Tyr Glu Gly Phe Tyr Leu Arg Gly Phe Phe Val
                580                 585                 590

Ala Lys Lys Arg Tyr Ala Val Ile Asp Glu Glu Gly Arg Ile Thr Thr
        595                 600                 605

Arg Gly Leu Glu Val Val Arg Arg Asp Trp Ser Glu Ile Ala Lys Glu
    610                 615                 620

Thr Gln Ala Lys Val Leu Glu Ala Ile Leu Lys Glu Asp Ser Val Glu
625                 630                 635                 640

Lys Ala Val Glu Ile Val Lys Asp Val Val Glu Glu Ile Ala Lys Tyr
                645                 650                 655

Gln Val Pro Leu Glu Lys Leu Val Ile His Glu Gln Ile Thr Lys Asp
            660                 665                 670

Leu Ser Glu Tyr Lys Ala Ile Gly Pro His Val Ala Ile Ala Lys Arg
        675                 680                 685

Leu Ala Ala Lys Gly Ile Lys Val Arg Pro Gly Thr Ile Ile Ser Tyr
    690                 695                 700

Ile Val Leu Arg Gly Ser Gly Lys Ile Ser Asp Arg Val Ile Leu Leu
705                 710                 715                 720

Ser Glu Tyr Asp Pro Lys Lys His Lys Tyr Asp Pro Asp Tyr Tyr Ile
                725                 730                 735

Glu Asn Gln Val Leu Pro Ala Val Leu Arg Ile Leu Glu Ala Phe Gly
            740                 745                 750

Tyr Arg Lys Glu Asp Leu Lys Tyr Gln Ser Ser Lys Gln Val Gly Leu
        755                 760                 765

Asp Ala Trp Leu Lys Lys
    770                 775

## Description

### FIELD OF THE INVENTION

Subject of the invention is a thermostable mutant B-type DNA-polymerase having a Y-GG/A amino acid motif between the N-terminal 3'-5'-exonuclease domain and the C-terminal polymerase domain in the wild type form whereas amino acids of this motif are substituted in the mutant form of the DNA polymerase and whereas these mutant DNA polymerases are suitable for PCR reactions. Thermostable mutants according to the present invention exhibit better performance in PCR reactions compared to the wild type DNA polymerase. A further embodiment of the present invention is the use of these thermostable mutants of the B-type DNA polymerase for polymerase chain reactions (PCR) and other nucleic acid synthesizing reactions. Another subject of the present invention is a method of producing the inventive mutants, vectors and cell lines comprising genes encoding the inventive mutants.

### BACKGROUND OF THE INVENTION

DNA-dependent DNA polymerases containing proof-reading activity have to coordinate two catalytic activities: the DNA polymerase activity and the exonuclease activity. For polymerase I type DNA polymerases (*E. coli* Pol I) as well as for B-type DNA polymerases, these catalytic activities are located on structurally distinct protein domains (Truniger, V., Lázaro, J., Salas, M. and Blanco, L. (1996) *EMBO J.*, 15(13), 3430-3441; Pisani, F. M., De Felice, M. and Rossi, M. (1998) *Biochemistry,* 37(42), 15005-15012). In B-type (eukaryotic-type) DNA polymerases, the coordination of the two catalytic activities was proposed to take place intramolecularly in the conserved motif Y-GG/A located between the N-terminal 3'-5' exonuclease and the C-terminal polymerization domain (Truniger, V., Lázaro, J., Salas, M. and Blanco, L. (1996) *EMBO J*., 15(13), 3430-3441; Pisani, F. M., De Felice, M. and Rossi, M. (1998) *Biochemistry,* 37(42), 15005-15012). For the Klenow fragment of *E.coli* DNA polymerase it was described, that the editing can be an intermolecular or intramolecular process involving dissociation and reassociation of the DNA depending on the local context (Joyce, C. M. (1989) *JBC,* 264(18), 10858-10866).
Truniger et al. (Truniger, V., Lázaro, J., Salas, M. and Blanco, L. (1996) *EMBO J.*, 15(13), 3430-3441) demonstrated for the mesophile replicative DNA polymerase ofbacteriophage φ29 that mutations in the Y-GG/A motif can lead to phenotypes favoring either polymerisation or exonucleolysis compared to the wild type enzyme. They could show that this effect is related to altered (ss)DNA binding parameters and that the motif is important for the communication between the polymerase and exonuclease active site in a combination of structural and functional roles.

For the DNA polymerase of the thermophilic crenarchaeon *Sulfolobus solfataricus (Sso)* a region of 70 amino acids (region 1) involved in enzyme-DNA interaction was determined (Pisani, F. M., Manco, G., Carratore, V. and Rossi, M. (1996) *Biochemistry,* 35, 9158-9166). It is located in the connecting part between the exonuclease domain and the polymerase domain and contains the Y-GG/A motif. By mutational analysis of the amino acids in the Y-GG/A motif, it could be shown that the amino acids in this part of the enzyme determine the processivity of the proofreading function (Pisani, F. M., De Felice, M. and Rossi, M. (1998) *Biochemistry,* 37(42), 15005-15012). Based on the crystal structure of bacteriophage RB69 DNA polymerase, Truniger et al. proposed a direct interaction of the tyrosine with the phosphodiester bond between the two nucleotides preceding the one acting as template (Truniger, V., Blanco, L. and Salas, M. (1999) *J. Mol. Biol.*, 286, 57-69).

### DESCRIPTION OF THE INVENTION

The subject of the present invention was to provide thermostable DNA polymerases exhibiting an improved performance in PCR. Especially, thermostable mutants of a B-type DNA polymerase are provided which exhibit improved PCR performance. The inventive mutants of the B-type DNA polymerase have mutations in the Y-GG/A amino acid motif. Preferred mutations refer to the position of the tyrosine in the Y-GG/A amino acid motif. Other mutations affecting the motif could also influence the performance of B-type DNA polymerases in PCR.

According to the present invention an improved performance of a DNA polymerase in PCR is defined as a performance that results in higher yields of PCR product, or the amplification of longer DNA targets. Additionally, improved PCR performance can be defined as improved fidelity during the amplification process.

Preferred mutant B-type DNA polymerases have mutations at the position of the tyrosine in the Y-GG/A amino acid motif. Preferred mutants of B-type DNA polymerases according to the present invention have phenylalanine, tryptophan or histidine at the position of the tyrosine. Other preferred mutants of B-type DNA polymerases according to the present invention have asparagine or serine at the position of the tyrosine. These mutant polymerases described here, in which the tyrosine of the Y-GG/A motif was substituted, exhibit an improved performance in PCR.

In a preferred embodiment the inventive mutant B-type DNA polymerase is a mutant of a B-type DNA polymerase obtainable from Euryarchaea, more preferrably from *Thermococcus aggregans (Tag).* Especially preferred is a mutant of a B-type DNA polymerase from *Tag* of about 94 kDa size with a temperature optimum of ≥80° C and the ability to perform polymerase chain reactions.

The present invention is described in detail for the B-type DNA polymerase from *Thermococcus aggregans*, but the invention could also be applied to other B-type DNA polymerases. Preferrably to those B-type DNA polymerases showing a high degree of homology (≥80%) to the DNA polymerase from *Thermococcus aggregans*.
The B-type DNA polymerase from *Thermococcus aggregans* exhibits a high degree of amino acid sequence homology to B-type DNA polymerases of other *Thermococcus* species. The homology of the DNA polymerases was calculated using the programm Blast 2 (Tatusova, T.A. and Madden, T.L. (1999) *FEMS Microbiol*. *Lett.* 174, 247 - 250). The homology of the B-type DNA polymerase from *Thermococcus aggregans* to the homologue enzymes from *Thermococcus* species is: 93% *(T. litoralis*), 87% *(T. gorgonarius*), 86% (*T. furiosus*) and 87% (T. spec. 9N7). The homology of the *Tag DNA* polymerase to polymerases from *Pyrococcus* species is: 86% *(P. abysii),* 86% (P. *horikoshii*), 86% (P. spec KOD) and 85% *(P. furiosus*). A lower homology is calculated to other B-type DNA polymerase from different euryarchaeota: 59% (*Methanococcus jannaschii*), 56% (*Methanococcus voltae*), 51% (*Methanobacterium thermoautotrophicum)* and 56% *(Archaeglobus fulgidus*). To B-type DNA polymerases from crenarchaeota and bacteriophages the homology is found as follows: 46% (*Sulfolobus solfataricus*), 42% (*Sulfolobus acidocaldarius),* 41% (*Sulfurisphera ohwakuensis*), 51% (*Aeropyrum pernix*), 40% (*Pyrodictium occultum),* 43% (*Cenarchaeum symbiosum),* 38% (bacteriophage T4) and 39% (bacteriophage RB69).

As described above several mutations in the Y-GG/A motif were performed for the *Sulfolobus solfataricus (Sso)* and the φ29 DNA polymerases. The observed effects on polymerase activity (pol) and exonuclease activity (exo) of these mutations do not completely correspond to the effects obtained for the *Tag* DNA polymerase. Thus the effect of the mutations on the performance of the mutants in PCR was not predictable.
The mutant Y387F of the *Tag* DNA polymerase exhibits a higher pol/exo ratio compared to the wild type *Tag* DNA polymerase. Similar results were described for *Sso* and φ29 DNA polymerase. The mutant G389A displays the opposite effect than the corresponding mutant in φ29 DNA polymerase: while G→A in *Tag* DNA polymerase almost knocks out polymerase activity, in φ29 DNA polymerase G→A mutant this activity is clearly enhanced. For mutants of the *Sso* DNA polymerase a change of exonuclease processivity was described. Again, this was not observed for mutants of the B-type *Tag* DNA polymerases. Thus, a prediction of the effect of analogous mutants in the Y-GG/A motif could not be made.

In summary, although it has been described in the prior art that the Y-GG/A motif plays a role in the coordination of the DNA polymerase activity and the exonuclease activity, the observed changes of the pol/exo ratio of the prior art DNA polymerases do not strictly correlate to the changes observed for the inventive mutants of the *Tag DNA* polymerase. Furthermore, it has not been described that the Y-GG/A motif is important for the performance of B-type DNA polymerase in PCR. Additionally, there is no correlation between the changes of the pol/exo ratio and the improvement of the performance of DNA polymerases in PCR. For instance, the mutant Y387H does not exhibit a change of pol/exo ratio compared to the wild-type, but it exhibits improved performance in PCR. Furthermore, a significant enhancement of fidelity was observed for the mutants Y387N and Y387S of *Tag* DNA polymerase.

Results obtained for the mutants of *Tag* DNA polymerase are described in more detail below.

### Enzymatic activities of wild type and mutant Tag DNA polymerases

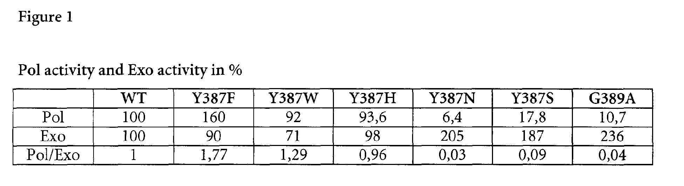

The enzymatic activities of the wild type enzyme and the mutants of *Tag* DNA polymerase were determined and analyzed (Figure 1). The DNA polymerase activity was determined as described in Example 2. According to the effect of the mutations on the polymerase activity three groups of mutants were defined: i) mutants with enhanced DNA polymerase activity (mutant Y387F), ii) mutants having a similar or slighlty reduced DNA polymerase activity compared to the wild type (mutants Y387W and Y387H) and iii) mutants with reduced DNA polymerase activity (mutants Y387N, Y387S, G389A).
The exonuclease activity was determined as described in Example 3. According to the effect of the mutations on the exonuclease activity two groups of mutants were defined: i) mutants with similar exonucleolytic activity as the wild type enzyme (mutants Y387F, Y387W, Y387H), ii) mutants with enhanced exonuclease activity (mutants Y387N, Y387S, G389A) in comparison to the wild type enzyme.

From the data obtained for polymerase activity and exonuclease activity the ratios of both activities (pol/exo) were calculated for the wild type enzyme and the mutants of *Tag* DNA polymerase (Figure 1). Three mutants showed a higher or similar pol/exo ratio as the wild type enzyme (mutants Y387F, Y387W, Y387H). Three mutants showed a clearly reduced pol/exo ratio in comparison to the wild type enzyme (mutants Y387N, Y387S, G389A).

### PCR performance

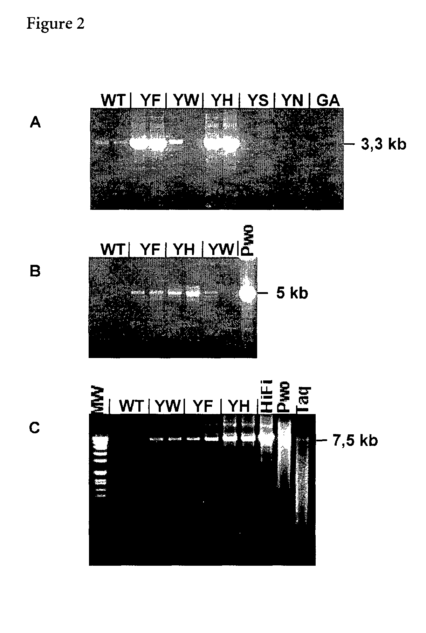

Wild type and mutant enzymes were submitted to polymerase chain reactions on lambda DNA in a buffer optimized for this purpose. All mutants except for mutant G389A were able to perform PCR, but yielded different amounts of product with a constant amount of enzyme (1 pmol). With increasing length of the DNA target, differences in performance of the enzyme were shown (Fig. 2). With 1 pmol of the mutants Y387S, Y387N and G389A no PCR product could be obtained for the amplification of a 3.3 kb fragment. 1 pmol of the wild type DNA polymerase could not amplify fragments of 5.0 kb length. The mutants Y387W, Y387F and Y387H were able to amplify a fragment of 7.5 kb length. As control *Taq* DNA polymerase, *Pwo* DNA polymerase and Expand™ High Fidelity PCR System (Roche Molecular Biochemicals) were used.

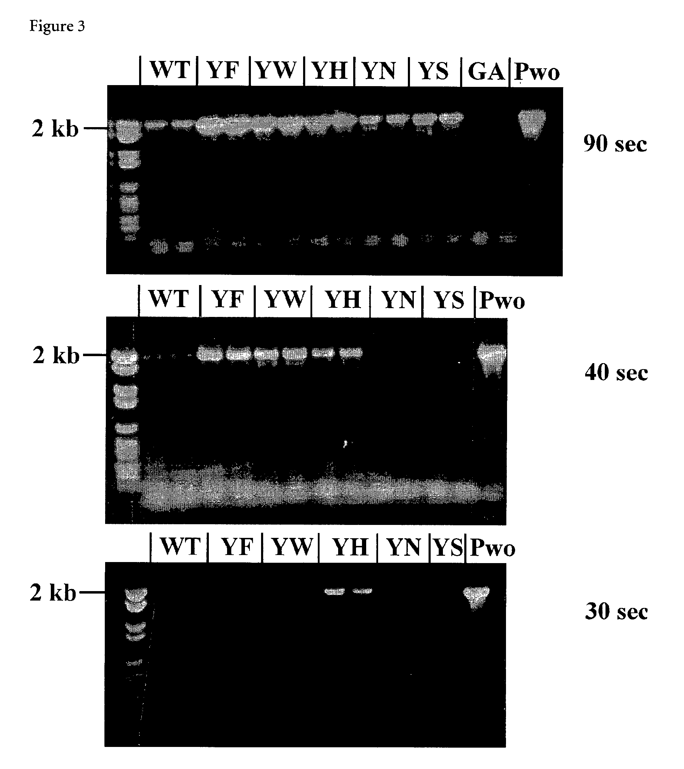

The differences in PCR performance were also shown by the amplification of a 2 kb fragment applying different elongation times in the PCR runs. Under these conditions, all enzymes tested except the mutant G389A were able to amplify a 2 kb fragment at an elongation time of 90 sec/cycle. The mutants Y387F, Y387W and Y387H were able to amplify the fragment at a reduced elongation time of 40 sec/cycle. The mutant Y387H was able to amplify the target in a elongation time of 30 sec/cycle (Fig. 3).

### Exonuclease processivity

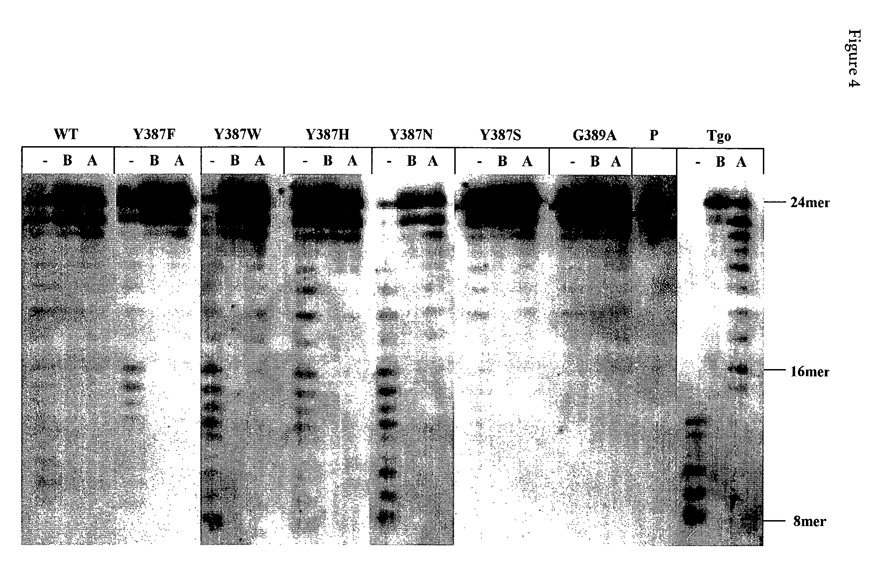

The exonuclease processivity of the enzymes was studied in an experiment based on the heparin trap method (Reddy, M. K., Weitzel, S. E. and von Hippel, P. H. (1992) *J. Biol. Chem.,* 267(20), 14157-14166; Pisani, F. M., De Felice, M. and Rossi, M. (1998) *Biochemistry,* 37(42), 15005-15012). A constant amount (1 pmol) of *Tag* DNA polymerase or its mutants was incubated for 4 minutes at 68 °C with a 5'-DIG-labelled 24mer oligonucleotide in the absence of nucleotides. In the absence of heparin, the oligonucleotide was continually degraded by the *Tag* enzymes (positive control, Fig. 4, lanes "-"). The function of the heparin trap method was demonstrated by addition of heparin and MnCl₂ before the binding of the enzyme (negative control, Figure 4, lanes B). Single turnover conditions (addition of heparin and MnCl₂ to start the reaction after the binding of enzyme) resulted in exonucleolytic degradation of the oligonucleotide by the *Tag* DNA polymerases (Fig. 4, lanes A). The enzymes showed differences in the exonucleolytic activity as shown by the different amounts of remaining oligonucleotide that was not degraded. However, for all enzymes tested the oligonucleotide was degraded to a similar extent (8 nt). This indicates a similar exonuclease processivity for the enzymes.
The *Thermococcus gorgonarius* DNA polymerase, which exhibits a strong exonuclease activity, was used as a positive control. It degraded the 24mer oligonucleotide in the absence of heparin to oligonucleotides of less than 15 bases length (Figure 4, lane "-"). Under single turnover conditions a strong degradation (11 nt) of the oligonucleotide is observed (Figure 4, lane "A").

### Fidelity

The error rates in amplification were determined for the mutant enzymes and the wild type DNA polymerase. The PCR-based fidelity assay described by Frey and Suppman (Frey, M. and Suppmann, B. (1995) *Biochemica*, 2, 34-35) was used. This method is based on the amplification, circulation and transformation of the pUC19 derivative pUCQ17, which contains a functional *lac*I^{q} allele (Barnes, W. M. (1994) *Proc. Natl. Acad. Sci. USA,* 91, 2216-2220). PCR-derived mutations in *lac*I result in a de-repression of the expression of *lac*Z*α* and subsequent formation of a functional β-galactosidase enzyme, which can be detected on X-Gal indicator plates.

In five independent runs, a mean error rate of 5.0x10⁻⁶ was found for the wild type *Tag DNA* polymerase. This value is in between the mean error rates of 1.8x10⁻⁶ for Expand™ High Fidelity PCR System (Roche Molecular Biochemicals) and 1.3x10⁻⁵ for *Taq* DNA polymerase (Roche Molecular Biochemicals) determined in the corresponding experiments. For a better comparison of the data, we plotted the quotient of the error rate determined for *Taq* DNA polymerase divided by the error rates determined for the *Tag* DNA polymerase and its mutants. In the independent experiments the error rate for *Taq* DNA polymerase varied from 1.2 to 3.05x10⁻⁵.

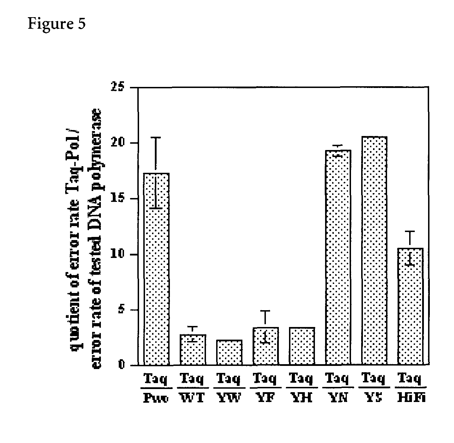

Figure 5 shows the quotients of the error rates of the wild type enzyme and mutants of *Tag* DNA polymerase. The error rates of the mutants showing improved PCR performance (Y387W, Y387F, Y387H) did not significantly differ from the values obtained for the wild type enzyme. The mutants with enhanced exonuclease activity (Y387N, Y387S) showed improved fidelity rates (Figure 5). For the mutants Y387N and Y387S mean error rates of 6.3x10⁻⁷ and 6.2x10⁻⁷ were determined.

In contrast to the φ29 DNA polymerase and the *Sso* DNA polymerase, the *Tag* DNA polymerase, is suited for PCR. The mutant enzymes (Y387F, Y387W, Y387H) with an aromatic amino acid in the position of the tyrosine showed a similar or only slightly enhanced DNA polymerase activity (mutants Y387F, Y387W, Y387H) but an improvement in PCR performance.

In the fidelity assay it was found that the mutants Y387F; Y387W and Y387H showed no significant change in their error rate. By contrast, the mutants Y387N or Y387S showed higher exonuclease activity and displayed an improved fidelity.

Subject of the present invention is also a method of producing the inventive B-type mutants comprising the following steps: cloning and mutagenesis of the gene, followed by the expression and purification of the protein.

Subject of the present invention is a DNA encoding for a thermostable B-type DNA polymerase having a Y-GG/A amino acid motif between the N-terminal 3'-5' exonuclease domain and the C-terminal polymerase domain in the wild type enzyme whereas the tyrosine of this motif is substituted in the mutant enzyme of the polymerase and whereas this mutant DNA polymerase is suitable for PCR.

Preferably, said DNA in wild type form is obtainable from Euryarchaea, more preferably from *Thermococcus aggregans (Tag).* Subject of the present invention is also a vector containing the inventive DNA. Suitable vectors are e.g. the following: pET14b/15b/16b/19b (Novagen); pRSET (Invitrogen); pTrcHis (Invitrogen); pHAT10/11/12 (Clontech); pPRO Tet.E/Lar.A (Clontech); pCALn/n-EK (Stratagene); pGEMEX-1/-2 (Promega).

Furthermore, subjects of the present invention are also cells comprising the above vector. Suitable cells are e.g. *E.coli* BL21, BL21(DE3), BL21(DE3)pLysS, BL21(DE3)pLysE, DH5∝PRO, JM109 (DE3), TOP10 in combination with the vectors recommended by the suppliers. The gene may have to be subcloned and the protein purification procedure may have to be adapted in the case of different expression vectors.
A sample of the recombinant strain expressing *Tag* DNA polymerase was deposited with the Deutsche Sammlung von Mikroorganismen und Zellkulturen (DSMZ) Mascheroder Weg 1b, D-38124 Braunschweig (DSM No. 13224).

A further subject of the invention is the use of the inventive mutant enzymes for synthesizing nucleic acids e.g. in PCR reactions.

### DETAILED DESCRIPTION OF THE PREFERED EMBODIMENTS

### Figures:

### Figure 1.

Table showing the relative polymerase activities (Pol) and 3'-5'-exonuclease activities (Exo) of *Tag* DNA polymerase and its mutants on double-stranded DNA.
Assays were carried out as described in example 2 and 3, respectiviely. The activites are expressed as percentage of the activity obtained for the wild-type *Tag DNA* polymerase.

### Figure 2.

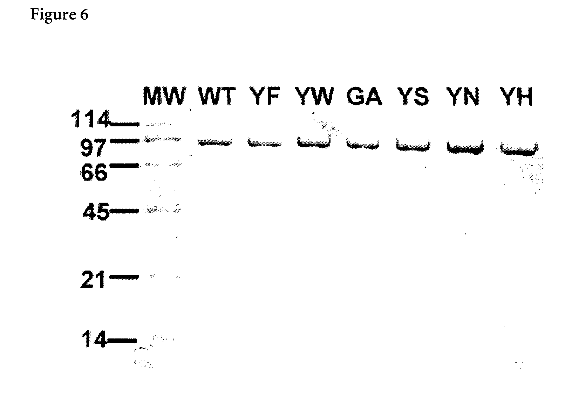

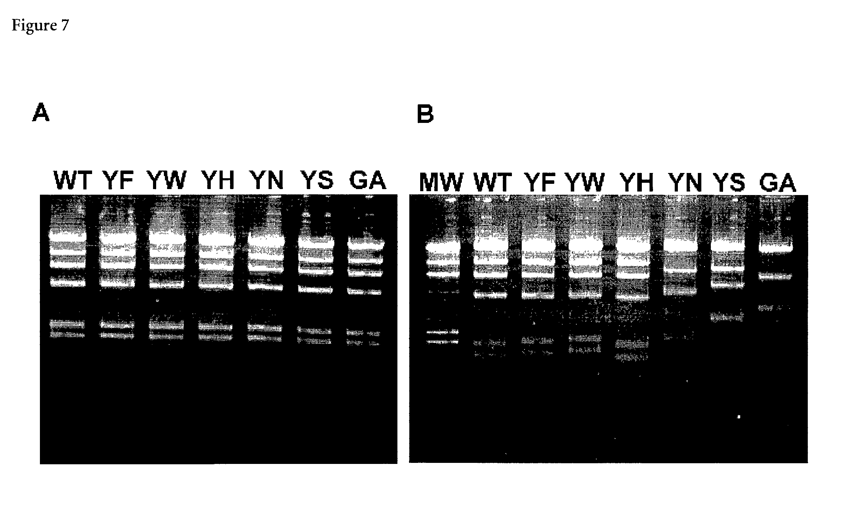

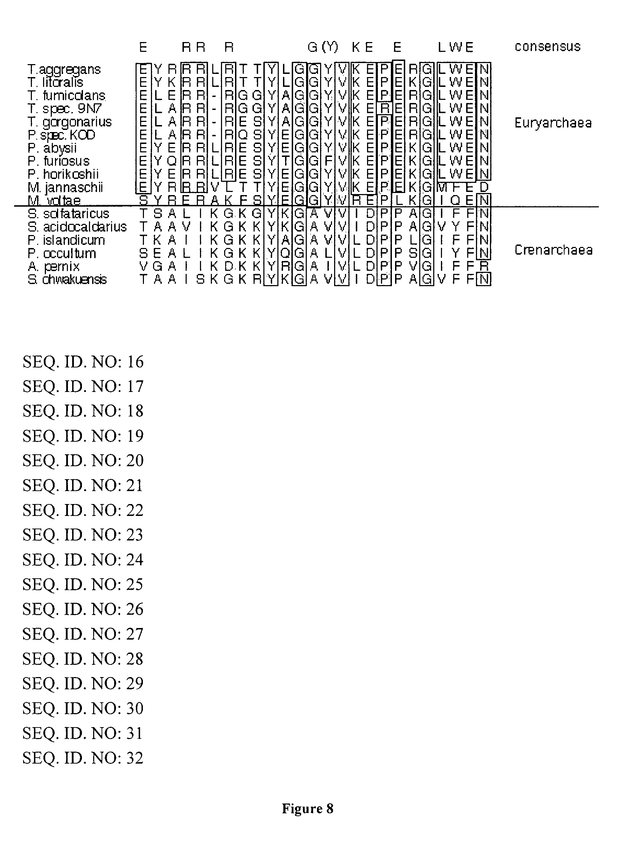

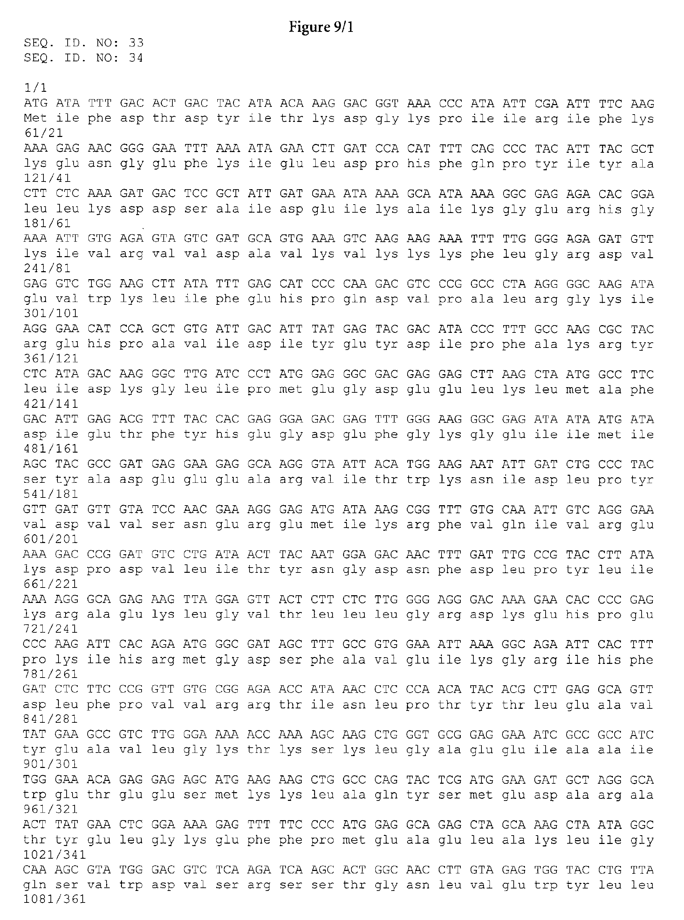

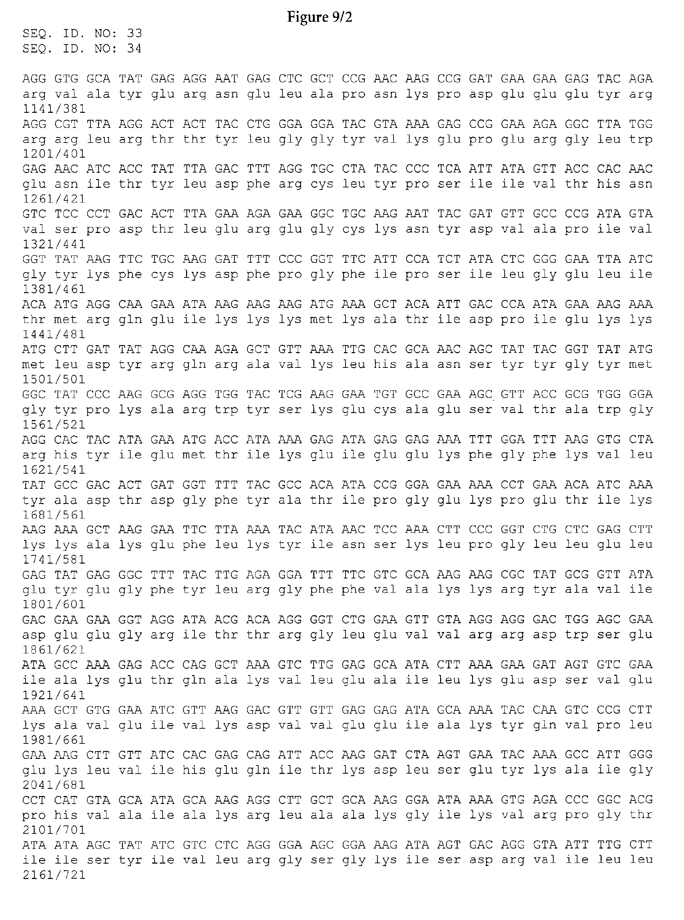

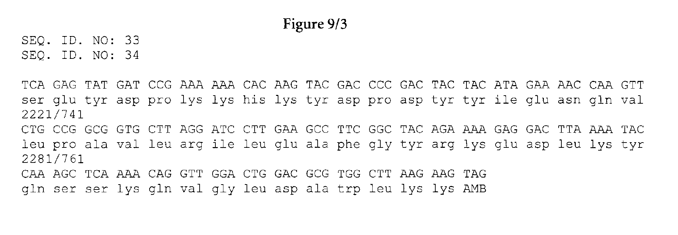

PCR with *Tag* DNA polymerase mutants.
*Tag* DNA polymerase mutants (1 pmol) were incubated in a 50 µl total volume with 10 ng of lambda DNA as template and 30 pmoles of a primer set designed to yield the indicated fragment lengths, 200 µM dNTPs and the suitable PCR buffer. Reactions were performed with 10 cycles of 10 sec 94 °C, 30 sec 57 °C and 3.0 min (A), 4.3 min (B) or 7.0 (C) min of elongation time at 72 °C followed by 20 cycles with elongation times increasing by 20 sec/cycle. After the PCR 5 µl sample were submitted to electrophoresis on a 1% agarose gel. For the control reaction 2.5 U of *Taq* DNA polymerase, *Pwo* DNA polymerase or Expand™ High Fidelity PCR System were used. The labeling of the lanes is described in the legend of figure 6.

### Figure 3.

Time-dependent polymerase chain reaction.
1% agarose gels showing 2 kb PCR products from reactions performed with different elongation time (90 sec, 40 sec, 30 sec as indicated) to determine the minimal elongation time.
1 pmol of each *Tag* DNA polymerase mutant or wild type enzyme was added to a mix of 10 ng lambda DNA and primers designed to yield 2 kb DNA fragments. Labeling of the lanes is described in the legend of figure 6. For each mutant duplicate reactions were performed. Right lane of each gel *Pwo:* 2.5 U *Pyrococcus woesei* DNA polymerase (Roche Molecular Biochemicals) as control reaction. Left lane of each gel: Molecular weight marker VI (Roche Molecular Biochemicals). In the 40 sec reaction, mutant GA was omitted. In the 30 sec reaction, for the mutant YS only one reaction was run on the gel.

### Figure 4.

3'-5'-exonuclease processivity.
The *Tag* DNA polymerase mutants tested are indicated on top of the figure. *Tgo* DNA polymerase was used as a control reaction (incubation for 30 sec). Reactions for wild type and mutants of *Tag* DNA polymerase were performed for 4 minutes at 68 °C after preincubation for 1 minute at 68°C. Lane "P" is the control reaction (24 mer 5'-DIG-labelled primer without incubation), lane "-": reaction without heparin (positive control); lane "B": reaction with heparin and MnCl₂ added before addition of the enzyme (negative control); lane "A": heparin and MnCl₂ added after the enzyme (reaction under single turnover conditions).

### Figure 5.

Fidelity of *Tag* DNA polymerase mutants.
The fidelity of *Tag* DNA polymerase and its mutants was expressed in relation to the fidelity of *Taq* DNA polymerase. A quotient of 1 means that the polymerase has the same error rate as *Taq* DNA polymerase (mean value 1.3x10⁻⁵). Values >1 reflect the factor by which a polymerase shows less errors than *Taq* DNA polymerase. The bars correspond to to mean values calculated from 2-5 independent experiments, error bars missing are smaller than 0.36. Abbreviations for enzymes are as indicated in legend to figure 6. As controls *Pyrococcus woesei* DNA polymerase (Roche Molecular Biochemicals) and Expand High Fidelity PCR System (Roche Molecular Biochemicals) were used ("Taq/Pwo" and "Taq/HiFi").

### Figure 6.

SDS-PAGE gel analysis of purified mutant proteins.
1 µg of each mutant was submitted to electrophoresis on an 10% SDS-PAGE gel. Left: MW, molecular weight marker; WT, *Thermococcus aggregans* wild type DNA polymerase; YF, YW, YS, YN, YH are the corresponding mutants with an exchange at the position of tyrosine 387 to phenylalanine, tryptophan, serine, asparagine, histidine, respectively. GA, mutation of glycine 389 to alanine in the gene of the *Thermococcus aggregans* DNA polymerase. All mutants showed the same chromatographic behaviour and solubility as the wild type enzyme.

### Figure 7.

Qualitative exonuclease assay.
A DNA molecular weight marker was used as substrate to test the exonucleolytic activity (DNA molecular weight marker II (MW II), Roche Molecular Biochemicals). 1 µg of MW II was incubated for 6 h at 65 °C with 1 pmol of each variant of *Tag* DNA polymerase in the presence (A) or absence (B) of 200 µM dNTP. *Tag* mutants are named as explained in legend of figure 6. Exonucleolytic degradation take place only in the absence of deoxynucleotides. The qualitative ranking of the proteins in terms of exonuclease activity is GA > YN > YS > YH > YF = YW = WT.

### Figure 8.

Consensus sequence motif for B-type DNA polymerases from the order of *Thermococcales* derived from a multiple alignment of amino acid sequences of euryarchaeal and crenarchaeal B-type DNA polymerases.
A region of 24 amino acids containing the Y-GG/A motif was analyzed with the ClustalW Software program (Higgins, EMBL Heidelberg, Germany). In addition to the amino acids conserved in all archaeal B-type DNA polymerases (like the Y-GG/A motif), a consensus sequence "E--RR-R-----G(Y)-KE-EE--LWE-" can be defined. This sequence is found in the sequence of all DNA polymerases belonging to the order of the *Thermococcales* and coincides with a homology of >80% of the DNA polymerases.
The sequences of the crenarchaeal species *Sulfolobus solfataricus*, *Sulfolobus acidocaldarius, Pyrobaculum islandicum*, *Pyrodictium occultum*, *Aeropyrum pernix*, *Sulfurisphaera ohwakuensis* and the sequences of several euryarchaeal species *Thermococcus ("T."), Pyrococcus ("P.")* and *Methanococcus* ("M.") were aligned.

### Figure 9.

DNA sequence and deduced amino acid sequence of recombinant wild type *Tag* DNA polymerase.
Three inteins found in the native gene (Acc. No. Y13030) were deleted by PCR (Niehaus, F., Frey, B., Antranikian, A. (1997) *Gene,* **204,** 153-158). Four mutations leading to amino acid exchanges were introduced during PCR. The amino acid exchanges (native→recombinant) are: L3F, A404T, S410C and L482H.

### Example 1

### Site-directed mutagenesis and expression of Tag DNA polymerase mutants

The cloning of the gene *of Tag DNA* Polymerase (polTY) was described earlier (Niehaus, F., Frey, B., Antranikian, A. (1997) *Gene,* **204,** 153-158). Overexpression of *Tag* DNA Polymerase in *E. coli* was achieved by subcloning its encoding gene into the IPTG-inducible pET15b vector (Novagen) containing an N-terminal His-Tag for purification (the resulting plasmid was named pET15b-TagPol).
The mutants presented in this study were prepared in polymerase chain reactions using primers containing the desired mutations as a mismatch. The forward primer was universally "Kpn-fw", matching to a sequence about 100 bp upstream of the mutation site and contained a *Kpn*I restriction site of the polTy gene. The reverse primers contained a *Sna*BI restrition site and additionally the desired mutation. The sequences of the oligonucleotides were as follows (mismatch sites for mutagenesis underlined): 
SEQ. ID. NO: 1
Kpn-Fw 5'-GCAACCTTGTAGAGTAGAGTGGTACCTGTTAAGGG-3';
 
SEQ. ID. NO: 2
TagY387F	5'-GCCTCTTTCCGGCTCTTTTACGTATCCTCCCAGGAAAGTAGTCC-3',
 
SEQ. ID. NO:3
TagY387H 5'-GCCTCTTTCCGGCTCTTTTACGTATCCTCCCAGGTGAGTAGTCC-3',
 
SEQ. ID. NO: 4
TagY387N 5'-GCCTCTTTCCGGCTCTTTACGTATCCTCCCAGGTTAGTAGTCC-3',
 
SEQ. ID. NO: 5
TagY387S 5'-GCCTCTTTCCGGCTCTTTACGTATCCTCCCAGGGAAGTAGTCC-3',
 
SEQ. ID. NO: 6
TagY387W 5'-GCCTCTTTCCGGCTCTTTACGTATCCTCCCAGCCAAGTAGTCC-3',
 
SEQ. ID. NO: 7
TagG389A 5'-GCCTCTTTCCGGCTCTTTTACGTATCCAGCCAGGTAAGTAGTCC-3'.
 PCR reactions were carried out with Expand™ High Fidelity PCR System (Roche Molecular Biochemicals) using the following program: 2 min 94 °C, 30 cycles of 10 sec 94 °C, 30 sec 55 °C, 30 sec at 72 °C. The resulting 139 bp fragments were digested with the restriction enzymes *Kpn*I and *Sna*BI yielding a 101 bp fragment that was ligated into pET15b-TagPol linearized with the restriction enzymes *Kpn*I and *Sna*BI. The cloned DNA fragments were sequenced to confirm the presence of the desired mutations.

For protein expression, *E. coli* BL21 (DE3) cells were transformed with the expression vector pET15b-TagPol. Three to five colonies were inoculated in 15 ml of LB medium supplemented with 100 µg Ampicillin per ml and grown to OD₆₀₀ₙₘ 0.3. An aliquot (10 ml) of the preculture was used to inoculate 500 ml of LB medium and incubated while shaking at 37°C. At OD₆₀₀ₙₘ= 0.6 expression was induced by addition of IPTG (final concentration: 1 mM). After incubation for 3 hours cells were harvested by centrifugation and suspended in 50 mM Tris-HCl/pH 7.5, 10 mM KCl, 0.5 mM EDTA, 4 mM MgCl₂, 5 mM DTT. Cells were sonicated on ice and the crude extract was heated for 15 min to 80 °C. Cell debris was removed by centrifugation (30 min, 30 000 x g at 4°C).
The supernatant was applied to a Blue Sepharose 3G-A column (Pharmacia) equilibrated with buffer A (50 mM Tris-HCl/ pH 7.5, 10 mM KCl, 4 mM Mg Cl₂). The protein was eluted with a gradient of 0.01-1.5 M KCl. Active fractions were pooled and dialyzed against 20 mM Tris-HCl /pH 7.9, 5 mM imidazole, 500 mM NaCl. The sample was applied to a Ni-chelate column (Novagene) equilibrated in the same buffer and eluted with a gradient of 0.005 - 1 M imidazole. Active fractions were pooled and dialyzed against storage buffer (50 mM Tris-HCl/pH 7.5, 100 mM KCl, 0.5 mM EDTA, 5 mM DTT, 50% glycerol). The enzymes were pure as shown by SDS gel electrophoresis (Figure 6).

### Example 2

### DNA polymerase assay

The DNA polymerase activity was determined by measuring the incorporation of α-(³²P)dCTP in a DNA substrate. The test mix (50 µl) contained 5 µl 10x *Tag* reaction buffer (100 mM Tris-HCl /pH 8.9, 750 mM KCl, 15 MgCl2, 100 mM CHAPS), 200 µM of each dATP, dGTP, dTTP, 100 µM dCTP, 1 mCi α-(³²P)dCTP, 1 µg of M13mp9 ssDNA annealed with 0.3 µg M13 primer. Assays were performed with 2 and 3 µl of enzyme in three different dilutions (final amount of enzyme 2.5 to 15 fmoles) yielding six reactions to calculate a mean value. As a reference *Pwo* DNA polymerase was used. The DNA/primer mix was prepared by heating 277.2 µg M13mp9 ssDNA (Roche Molecular Biochemicals) and 156 µg M13 sequencing primer (17mer forward primer, Roche Molecular Biochemicals) for 30 minutes to 55 °C and then cooling it for 30 minutes to room temperature.

Assay reactions were incubated for 30 minutes at 65 °C, stopped on ice by addition of 500 µl of 10% TCA (4 °C) and kept on ice for another 10 minutes. Samples were filtered over GFC-filter (Whatman), filters washed three times with 5% TCA, dried and submitted to β-counting in 2 ml of scintillation fluid. One unit is defined as the amount of enzyme necessary to incorporate 10 nM dNTP into acid insoluble material at 65 °C in 30 minutes.

### Example 3

### Exonuclease Assays

### Activity Assay

3 µl (300 ng) of enzyme (approximately 5 Units of polymerase activity) were incubated with 5 pg of 3H-labelled calf thymus DNA for 4 hours at 65 °C in a buffer containing 10 mM Tris-HCl/pH 8.9, 75 mM KCl, 1.5 MgCl₂, 10 mM CHAPS. Radioactivity liberated from calf thymus DNA was measured in a scintilation counter.
The assay used does not discriminate between the 3'-5' exonuclease activity and the 5'-3' exonuclease activity. 5'-3'-exonuclease activity has not been detected pheno- or genotypically in B-type polymerases of *Thermococcales* (Perler, F. B., Kumar, S. and Kong, H. (1996) *Adv. Prot. Chem.,* **48**, 377-435). Thus the values obtained can be regarded as 3'-5'-exonuclease activity.

In another assay, 1 µg of molecular weight marker II (Roche Molecular Biochemicals) were incubated in same buffer as above with 5 U of the indicated protein with or without 200 µM dNTP in a final volume of 50 µl for 6 hours at 65 °C. The reaction products were separated by electrophoresis on 1% agarose gels.

### 3'-5' Exonuclease processivity assay

The previously described heparin trap method was used (Reddy, M. K., Weitzel, S. E. and von Hippel, P. H. (1992) *J. Biol. Chem.*, **267**(20), 14157-14166; Pisani, F. M., De Felice, M. and Rossi, M. (1998) *Biochemistry,* 37(42), 15005-15012). A reaction mix (10 µl) containing 10 mM Tris-HCl/pH 8.9, 75 mM KCI, 10 mM CHAPS and 0.5 pmoles of a 5'-DIG-labeled 24mer oligonucleotide was prewarmed for 1 minute at 68 °C in a thermocycler. Unless otherwise noted, 1 pmol of the enzyme was preincubated for 1 minute at 68 °C with the substrate. The reaction was started by addition of MnCl₂ (final concentration: 4 mM) and heparin (final concentration:1 mg/ml) to ensure single turnover conditions. After incubation for 4 minutes, the reaction was stopped by the addition of 5 µl of formamide buffer (80% formamide, 10 mM EDTA, 1 mg/ml bromophenol blue, 1 mg/ml xylene xyanol). The efficiency of the heparin trap was checked in a control reaction by adding heparin and MnCl₂ prior to the addition of the enzyme. The samples were denaturated for 3 minutes at 90 °C and subjected to denaturing gel electrophoresis on a 17.5 % polyacrylamide/8 M urea gel. Gels were blotted to a positively charged nylon membrane (Roche Molecular Biochemicals) and the blots developed with CPD-Star (Roche Molecular Biochemicals) according to the manufacturers instructions.

### Example 4

### lacI-based PCR fidelity assay

We used the *lac*I-based PCR fidelity assay described by Frey and Suppmann (Frey, M. and Suppmann, B. (1995) *Biochemica*, 2, 34-35). This method is based on the amplification, circularization and transformation of the pUC19 derivative pUCQ17, which contains a functional *lac*I^{q} allele (Barnes, W. M. (1994) *Proc. Natl*. *Acad. Sci. USA,* **91**, 2216-2220). PCR-derived mutations in *lac*I result in a de-repression of the expression of *lac*Z*α* and subsequent formation of a functional β-galactosidase enzyme, which can be easily detected on X-Gal indicator plates.

The truncated *lac*I gene of pUC19 was substituted by a functional copy of *lac*I^{q}. A 178 bp *Pvu II-Afl III* fragment was replaced by a 1121 bp DNA fragment encoding *lac*I^{q}. The α-complementing *E. coli* strain DH5α, once transformed with the resulting plasmid pUCIQ17 (3632 bp), produces white (LACI⁺) colonies on LB plates containing ampicillin (100 µg/ml) and X-Gal (0,004% w/v). For the PCR, pUCIQ17 was linearized by digestion with *Dra* II and used as a template in an amount of 1 or 10 ng. Both primers have *Cla I* cleavage sites at their 5' ends. Oligonucleotide Cla33 (34mer, 24 matches: SEQ. ID. NO: 8: 5'-AGC TTA TCG ATG GCA CTT TTC GGG GAA ATG TGC G-3') and Oligonucleotide Cla55 (36mer, 26 matches: SEQ. ID. NO: 9: 5'- AGC TTA TCG ATA AGC GGA TGC CGG GAG CAG ACA AGC-3') resulted in a PCR product of 3493 bp. The reactions were performed with 1 or 5 pmol of protein in the *Tag* polymerase PCR buffer described below or for the control reactions in the manufacturers PCR buffers with 2.5 U of enzyme. The cycle conditions were 10 sec denaturation at 94 °C, 30 sec annealing at 57 °C and 4 min elongation at 72 °C for 18, 24 or 30 cycles depending on the enzyme.
After PCR, the yield of amplification product was determined at (OD₂₆₀ₙₘ or in agarose gel) and the DNA fragments submitted to phenol/chloroform extraction to eliminate any protein. After digestion with *ClaI*, the DNA fragments were purified from a preparative agarose gel. Ligation reactions were carried out with the Rapid Ligation Kit (Roche Molecular Biochemicals), the reactions contained 30 ng DNA. The resulting circular plasmids were transformed in *E. coli* DH5α as described by Hanahan (Hanahan, D. (1983) *J. Mol. Biol.*, 166, 557-580) and plated on LB Amp/X-Gal plates described above. After incubation overnight at 37 °C, blue and white colonies were counted. The error rate (f) per bp was calculated with a rearranged equation published by Keohavong and Thilly (Keohavong, P. and Thilly, W. G. (1989) *Proc. Natl. Acad.*
*Sci. USA,* 86, 9253-9257): f = -lnF / d x b bp.

Where F is the fraction of white colonies (white colonies/ total colonies); **d** is the number of DNA duplications: 2^{d} = output DNA/input DNA and b is the effective target size (1080 bp) of the *lac*I gene. There are 349 phenotypically identified (by colour screening) single-base substitutions (non-sense and mis-sense) at 179 codons (approximately 50% of the coding region) within the *lac*I gene. (Provost, G. S., Kretz, P. L., Hamner, R. T., Matthews, C. D., Rogers, B. J., Lundberg, K. S., Dycaico, M. J. and Short, J. M. (1993) *Mut. research,* **288,** 133-149). Frameshift errors which may occur at every position in the 1080 bp open reading frame of *lac*I, are not taken into account because little information is available for the specific polymerases used in PCR systems except for *Taq* DNA polymerase.

### Example 5

### Polymerase Chain Reactions

PCR was performed in a buffer optimized for *Tag* DNA polymerase and its mutants: 10 mM Tris-HCl/pH 8.9, 75 mM KCl, 1.5 MgCl₂, 10 mM CHAPS, 200 µM dNTP. 10 ng of λ DNA were used as a template and 30 pmol of each primer (20 bp, designed to yield the products of the desired length): 
Lambda 1, universal forward primer: 5'-GAT GAG TTC GTG TCC GTA CAA CA-3', SEQ. ID.
NO: 10,
 Lambda 3.3: 5'-CTC ATC AGC AGA TCA TCT TCA GG-3', SEQ. ID. NO: 11, Lambda 8: 5'-ACT CCA GCG TCT CAT CTT TAT GC-3', SEQ. ID. NO: 12, Lambda 9: 5'-GAT GGT GAT CCT CTC TCG TTT GC-3', SEQ. ID. NO: 13. Lambda 3.3, 8 and 9 were used as reverse primers for the amplification of 3.3 kb, 5 kb and 7.5 kb fragments respectively.
Template, primers and nucleotides were prepared in mix 1 in a volume of 25 µl. Then 25 µl of mix 2 containing the buffer and enzyme (1 pmol *Tag* wild type or mutant; or 2.5 U control enzyme) were added. All reactions were prepared in duplicate. The amplification was performed in a 2400 GeneAmp thermocycler (Perkin Elmer). The cycle conditions were: 2 min at 94 °C, 10 cycles with 10 sec denaturation at 94 °C, 30 sec annealing at 58 °C and elongation at 72 °C.

Elongation times depended on the length of the product (3 min for 3.3 kb, 4.3 min for 5 kb and 7 min for 7,5 kb). Another 20 cycles were performed with increasing the elongation times by 20 sec/cycle. The reaction was finished by 7 minutes at 72 °C. The tubes were kept at 4°C until separation by electrophoresis on 1% agarose gels.

The improvement of the PCR performance of the mutants was also studied in a "time-dependent PCR". A 2 kb fragment was amplified from lambda DNA as described above. In these studies the elongation time of the PCR was stepwise reduced (90 sec, 40 sec, 30 sec) to determine for each enzyme the minimal elongation time that was sufficient to amplify the 2 kb fragment. The following primers were used:
Lambda 1, universal forward primer: 5'-GAT GAG TTC GTG TCC GTA CAA CA-3', SEQ. ID. NO: 14,
Lambda 6, reverse primer: 5'-CTT CAT CAT CGA GAT AGC TGT CG-3', SEQ. ID. NO: 15.
The temperature profile was as described above. The elongation times were kept constant over 30 cycles.

## Claims

1. A thermostable mutant B-type DNA polymerase having a Y-GG/A amino acid motif between the N-terminal 3'-5'-exonuclease domain and the C-terminal polymerase domain in the wild-type form of the polymerase whereas preferrably the tyrosine of this motif is substituted in the mutant form of the polymerase and whereas this mutant DNA polymerase is suitable for polymerase chain reactions.

2. A mutant B-type DNA polymerase according to claim 1 having an amino acid with an aromatic side chain at the position of the tyrosine.

3. A mutant thermostable B-type DNA polymerase according to claim 1 having a Y→F,Y→W or Y→H mutation.

4. A mutant B-type DNA polymerase according to claim 1 having an amino acid with an hydrophilic side chain at the position of the tyrosine.

5. A mutant thermostable B-type DNA polymerase according to claim 1 having a Y→N or Y→S mutation.

6. A mutant thermostable B-type DNA polymerase according to claims 1-5 whereas the wild type form is obtainable from Euryarchaea.

7. A mutant thermostable B-type DNA polymerase according to claims 1-6 whereas the wild type form is obtainable from *Thermococcus aggregans*.

8. A mutant of a thermostable B-type DNA polymerase according to claims 1-6 whereas the amino acid sequence of the wild type form is ≥80% homologue to the amino acid sequence of wild type *Tag* DNA polymerase.

9. A DNA encoding a thermostable mutant DNA polymerase according to claims 1-8.

10. A vector containing a DNA according to claim 9.

11. A transformed host cell comprising a vector according to claim 10.

12. A process for obtaining a polymerase according to claim 1-8 comprising the steps of cloning and mutagenesis of the gene, followed by the expression and purification of the protein.

13. Use of a polymerase according to claim 1-8 for synthesizing nucleic acids.

14. Use of a polymerase according to claims 1-8 for PCR reactions.
